# EUROPEAN PATENT SPECIFICATION

(11) **EP 1 339 458 B1**
(45) Date of publication and mention of the grant of the patent: **15.08.2007**
(21) Application number: 01997301.5
(22) Date of filing: 20.11.2001
(51) Int. Cl.: A61P 35/00, A61K 45/06, A61K 31/505, A61K 31/70

(54) **COMBINATION COMPRISING AN AGENT DECREASING VEGF ACTIVITY AND AN AGENT DECREASING EGF ACTIVITY**
KOMBINATION ENTHALTEND EIN MITTEL ZUR VERMINDERUNG VON VEGF-AKTIVITÄT UND EIN MITTEL ZUR VERMINDERUNG VON EGF-AKTIVITÄT
COMBINAISON COMPRENANT UN AGENT DIMINUANT L'ACTIVITE DU FACTEUR DE CROISSANCE ENDOTELIAL VASCULAIRE ET UN AGENT DIMINUANT L'ACTIVITE DU FACTEUR DE CROISSANCE DE L'EPIDERME

(30) Priority: 22.11.2000 GB 0028467; 10.09.2001 GB 0121813
(43) Date of publication of application: 03.09.2003
(60) Divisional application: 07108093.1
(73) Proprietor: Novartis AG, 4056 Basel (CH); Novartis Pharma GmbH, 1235 Vienna (AT)
(72) Inventor: WOOD, Jeanette, Marjorie, CH-4105 Biel-Benken (CH); BRANDT, Ralf, Greenwith, SA 5125 (AU); BOLD, Guido, CH-5073 Gipf-Oberfrick (CH); TRAXLER, Peter, CH-4124 Schönenbuch (CH)
(74) Representative: Gros, Florent
(86) International application number: PCT/EP2001/013441
(87) International publication number: WO 2002/041882

(56) References cited:
- WO-A-00/00206
- WO-A-00/59509
- WO-A-01/32651
- WO-A-01/47507
- WO-A-96/33980
- WO-A-98/35958
- US-A- 5 731 325
- SHAHEEN R M ET AL: "Inhibited growth of colon cancer carcinomatosis by antibodies to vascular endothelial and epidermal growth factor receptors" BRITISH JOURNAL OF CANCER, vol. 85, no. 4, 17 August 2001 (2001-08-17), pages 584-589, XP008052140 ISSN: 0007-0920

## Description

The invention relates to a combination which comprises a first active ingredient which is a vasculostatic compound, preferably a compound which decreases the activity of the vascular endothelial growth factor (VEGF), and a second active ingredient which decreases the activity of the epidermal growth factor (EGF), especially for the delay of progression or treatment of a disease associated with deregulated angiogenesis, in particular a proliferative disease; a pharmaceutical composition comprising such a combination; the use of such a combination for the preparation of a medicament for the delay of progression or treatment of a proliferative disease; a commercial package or product comprising such a combination as a combined preparation for simultaneous, separate or sequential use; the use of a vasculostatic compound in combination with a compound which decreases the activity of the EGF.

The use of vasculostatic compounds for the treatment of proliferative diseases is already known in the art. At the centre of the network regulating the growth and differentiation of the vascular system and its components, both during embryonic development and normal growth and in a wide number of pathological anomalies and diseases, lies the angiogenic factor known as "Vascular Endothelial Growth Factor", along with its cellular receptors (see Breier, G., et al., Trends in Cell Biology 6, 454-6 [1996] and references cited therein). VEGF is a dimeric, disulfide-linked 46-kDa glycoprotein. VEGF receptors are transmembranous receptor tyrosine kinases. They are characterized by an extracellular domain with seven immunoglobulin-like domains and an Intracellular tyrosine kinase domain. Certain diseases are known to be associated with deregulated angiogenesis, for example diseases caused by ocular neovascularisation, such as retinopathies (including diabetic retinopathy), age-related macula degeneration, psoriasis, haemangioblastoma, haemangioma, arteriosclerosis, an inflammatory disease, such as a rheumatoid or rheumatic Inflammatory disease, especially arthritis, such as rheumatoid arthritis, or other chronic inflammatory disorders, such as chronic asthma, arterial or post-transplantational atherosclerosis, endometriosis, and especially proliferative diseases, for example so-called solid tumours and liquid tumours (such as leukaemias).

A large number of human tumors, especially gliomas and carcinomas, express high levels of VEGF and its receptors. Direct evidence of the role of VEGF as a tumor angiogenesis factor *in vivo* has been obtained from studies in which VEGF expression or VEGF activity was inhibited. This was achieved with antibodies which inhibit VEGF activity, with dominant-negative VEGFR-2 (also called KDR) mutants which inhibited signal transduction, or with the use of antlsense-VEGF RNA techniques. All approaches led to a reduction in the growth of glioma cell lines or other tumor cell lines *in vivo* as a result of inhibited tumor angiogenesis.

The tyrosine kinase activity of the receptor for epidermal growth factor (EGF) plays a key role in signal transmission in a large number of mammalian cells, including human cells, especially epithelial cells, cells of the immune system and cells of the central and peripheral nervous system. For example, in various cell types, EGF-induced activation of receptor-associated tyrosine protein kinase (EGF-R-TPK) is a prerequisite for cell division and hence for the proliferation of the cell population. An increase in the number of EGF-receptor-specific tyrosine kinase inhibitors thus inhibits the proliferation of the cells.

Compounds which inhibit the tyrosine kinase activity of the receptor for the epidermal growth factor are therefore useful, for example, in the treatment of benign or malignant tumours. They are capable of preventing the formation of tumour metastases and the growth of micrometastases. They can be used especially in the case of epidermal hyperproliferation (psoriasis), in the treatment of neoplasias of epithelial character, e.g. mammary carcinomas, and in leukaemias. Such compounds can also be used in the treatment of disorders of the central or peripheral nervous system in which signal transmission by several or, especially, a single tyrosine protein kinase(s) and/or serine/threonine protein kinase(s) is/are involved.

A large number of VEGF and EGF tyrosine kinase activity inhibitors have been described in the art. Also VEGF and EGF receptor inhibitors and compounds binding to VEGF or EGF, e.g. antibodies, are known. In the case of a proliferative disease in general the maximum effect that can be achieved with these agents is in most cases a stable disease, i.e. tumorstasis. Side-effect known for EGF receptor tyrosine kinase inhibitors are diarrhea and skin rashes.

Surprisingly, it has now been found that the anti-proliferative effect of a combination which comprises a first active ingredient which is a vasculostatic compound, i.e. a compound which decreases the activity of the VEGF, and a second active ingredient which decreases the activity of the EGF, is greater than the maximum effect that can be achieved with either type of ingredient as monotherapy.

Hence, the invention relates to a combination, such as a combined preparation or pharmaceutical composition, which comprises a first active ingredient which is a vasculostatic compound and a second active ingredient which decreases the activity of the EGF, in which the active ingredients are present in each case in free form or in the form of a pharmaceutically acceptable salt and optionally at least one pharmaceutically acceptable carrier; for simultaneous, separate or sequential use. The first active ingredient is a compound which decreases the activity of the VEGF. Such combination can be used for the delay of progression or, preferably, the treatment of a disease associated with deregulated angiogenesis, in particular a proliferative disease, and especially a proliferative disease which responds to the treatment with the single active ingredients.

As disclosed herein, in many cases tumor regression is observed upon treatment of the tumor with such a combination.

The active ingredient which decreases the activity of the VEGF is selected from the group consisting of compounds which inhibit the VEGF receptor tyrosine kinase, compounds which inhibit a VEGF receptor and compounds binding to VEGF.

The second active ingredient which decreases the activity of the epidermal growth factor EGF is selected from the group consisting of compounds which inhibit the EGF receptor tyrosine kinase, compounds which inhibit the EGF receptor and compounds binding to EGF.

A number of peptides are reported to effect the activity of the VEGF or the EGF. Peptides have the disadvantage to get easily hydrolyzed under physiological conditions, especially those physiological conditions to be found in the blood or stomach of warm-blooded animals. Therefore, such compounds in the present invention are no peptides.

The potency of the compound to inhibit a tyrosine kinase, e.g., VEGF or EGF tyrosine kinase, can, e.g., be evaluated by incubating compounds with the tyrosine kinase in the presence of [³³P]-ATP and an artificial substrate, using optimised buffer and salt conditions. Phosphorylated tyrosine on the substrate is then detected by means of a β-scintillation counter. The drug concentration required to inhibit the VEGF or EGF enzyme activity by 50 % (IC50 value) of compounds which inhibit a VEGF or the EGF receptor tyrosine kinase as defined herein is typically between 10 and 150 nM, preferably between 15 and 50 nM.

The "Compound which inhibits a VEGF receptor tyrosine kinase" as defined herein is a compound which interacts more strongly with at least one VEGF receptor tyrosine kinase than with the EGF receptor tyrosine kinase. Preferably, the interaction with the VEGF receptor tyrosine kinase is at least 4-fold, more preferably at least 10-fold and most preferably at least 50-fold, stronger than the interaction with the EGF receptor tyrosine kinase.

The "Compound which inhibit the EGF receptor tyrosine kinase" as defined herein is a compound which interacts more strongly with the EGF receptor tyrosine kinase than with the VEGF receptor tyrosine kinase. Preferably, the interaction with the EGF receptor tyrosine kinase is at least 4-fold, more preferably at least 10-fold and most preferably at least 50-fold, stronger than the Interaction with the VEGF receptor tyrosine kinase.

The "Compound which inhibits a VEGF receptor" as defined herein interacts more strongly with a VEGF receptor than with the EGF receptor. The compound binding to VEGF as defined herein interacts more strongly with VEGF than with EGF. Preferably, in both cases the interaction with a VEGF receptor or VEGF is at least 4-fold, more preferably at least 10-fold and most preferably at least 25-fold, stronger than the interaction with the EGF receptor tyrosine kinase or EGF, respectively.

The "Compound which inhibits an EGF receptor" as defined herein interacts more strongly with an EGF receptor than with the VEGF receptor. The Compound binding to EGF as defined herein interacts more strongly with EGF than with VEGF. Preferably, In both cases the interaction with an EGF receptor or EGF is at least 4-fold, more preferably at least 10-fold and most preferably at least 25-fold, stronger than the interaction with the VEGF receptor tyrosine kinase or VEGF, respectively.

The structure of the active ingredients identified by code nos., generic or trade names may be taken from the actual edition of the standard compendium "The Merck Index" or from databases, e.g. Patents international (e.g. IMS World Publications). Any person skilled in the art is fully enabled to identify the active ingredients and, based on these references, likewise enabled to manufacture and test the pharmaceutical indications and properties in standard test models, both *in vitro* and *in vivo.*

The term "a combined preparation", as used herein defines especially a "kit of parts" in the sense that the first and second active ingredient as defined above can be dosed independently or by use of different fixed combinations with distinguished amounts of the ingredients, i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub-population to be treated or the needs of the single patient which different needs can be due to age, sex, body weight, etc. of the patients. There is at least one beneficial effect, e.g., a mutual enhancing of the effect of the first and second active ingredient, in particular a synergism, e.g. a more than additive effect, additional advantageous effects, less side effects, a combined therapeutical effect in a non-effective dosage of one or both of the first and second active Ingredient, and especially a strong synergism the first and second active ingredient.

The term "delay of progression" as used herein means administration of the pharmaceutical combination to patients being in a pre-stage of a disease associated with deregulated angiogenesis, especially a proliferative disease, to be treated, in which patients a pre-form of the corresponding disease is diagnosed or which patients are in a condition, e.g., during a medical treatment or a condition resulting from an accident, under which it is likely that a corresponding disease will develop.

The term "a disease associated with deregulated angiogenesis" relates especially to diseases caused by ocular neovascularisation, especially retinopathies, such as diabetic retinopathy or age-related macular degeneration, psoriasis, haemangioblastoma, such as haemangioma, mesangial cell proliferative disorders, such as chronic or acute renal diseases, e.g. diabetic nephropathy, malignant nephrosclerosis, thrombotic microangiopathy syndromes or transplant rejection, or especially inflammatory renal disease, such as glomerulonephritis, especially mesangioproliferative glomerulonephritis, haemolytic-uraemic syndrome, diabetic nephropathy, hypertensive nephrosclerosis, atheroma, arterial restenosis, autoimmune diseases, acute inflammation, fibrotic disorders (e.g. hepatic cirrhosis), neurodegenerative disorders and especially proliferative diseases (solid tumours, but also leukemias and other "liquid tumours", especially those expressing c-kit, KDR or fit-1), such as especially breast cancer, cancer of the colon and generally the GI tract, cervix cancer, e.g. glioma, ovarian cancer, lung cancer, especially small-cell lung cancer, but also non-small-cell lung cancer and mesothelioma, head and neck cancer, skin cancer, in particular squamous cell carcinoma of the skin, bladder cancer, renal cancer, cancer of the prostate, especially hormone refractory prostate cancer, or Kaposi's sarcoma. The combinations disclosed herein inhibit the growth of tumours and are especially suited to prevent the metastatic spread of tumours and the growth of micrometastases.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

A pharmaceutical combination which comprises a vasculostatic compound and a second active ingredient which decrease the activity of the EGF, in which the active ingredients are present in each case in free form or in the form of a pharmaceutically acceptable salt, if at least one salt-forming group is present, will be referred to hereinafter as a COMBINATION OF THE INVENTION.

The nature of proliferative diseases is multifactorial. Under certain circumstances, drugs with different mechanisms of action may be combined. However, just considering any combination of drugs having different mode of action but acting in the similar field does not necessarily lead to combinations with advantageous effects.

All the more surprising is the experimental finding that the administration of a COMBINATION OF THE INVENTION results not only in a beneficial, especially a synergistic, therapeutic effect documented, e.g., by an increased rate in overall survival, but also in further surprising beneficial effects, e.g. less side effects, compared to a monotherapy applying only one of the pharmaceutically active ingredients used in the COMBINATION OF THE INVENTION.

It can be shown by established test models, e.g. *in vivo* tests against NCI H-596 human small cell lung tumors or NeuT-driven genetically engineered mouse breast tumors, or a clinical study, and especially those test models and studies described herein, that a COMBINATION OF THE INVENTION results in a more effective delay of progression or treatment of a proliferative disease compared to the effects observed with the single active ingredients. The person skilled In the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter. Such clinical studies are preferably randomized, double-blind, clinical studies in patients with advanced carcinoma. Such studies demonstrate, in particular, the synergism of the active ingredients of the COMBINATIONS OF THE INVENTION. The beneficial effects on proliferative diseases can be determined directly through the results of these studies or by changes in the study design which are known as such to a person skilled in the art. The studies are, in particular, suitable to compare the effects of a monotherapy using the active ingredients and a COMBINATION OF THE INVENTION. The efficacy of the treatment is determined In these studies, e.g., after 18 or 24 weeks by radiologic evaluation of the tumors every 6 weeks with the control achieved on monotherapy with one of both active ingredients plus a placebo matching with the second of both active ingredients. The patients are treated with the COMBINATION OF THE INVENTION or one of both active ingredients, e.g., once every three weeks.

A further benefit is that lower doses of the active ingredients of the COMBINATION OF THE INVENTION can be used, for example, that the dosages need not only often be smaller but are also applied less frequently, or can be used in order to diminish the incidence of side effects. This is in accordance with the desires and requirements of the patients to be treated.

It is one objective of this invention to provide a pharmaceutical composition comprising a quantity, which is jointly therapeutically effective against a disease associated with deregulated angiogenesis, comprising a vasculostatic compound or a pharmaceutically acceptable salt thereof and a second active ingredient or a pharmaceutically acceptable salt thereof which decrease the activity of the EGF, and at least one pharmaceutically acceptable carrier. In this composition, the first and second active ingredient can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active ingredient, alone or in combination with one or more pharmaceutically acceptable carries, especially suitable for enteral or parenteral application. The preferred route of administration of the dosage forms of the present invention is orally.

The novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of active ingredient or ingredients contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, for example, water, glycols, oils, alcohols, flavoring agents, preservatives, coloring agents; or carriers such as starches, sugars, microcristalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed.

Furthermore, the present invention relates to the use of a COMBINATION OF THE INVENTION for the preparation of a medicament for the delay of progression or treatment of a disease associated with deregulated angiogenesis.

Moreover, the present invention provides a commercial package comprising as active ingredients COMBINATION OF THE INVENTION, together with instructions for simultaneous, separate or sequential use thereof in the delay of progression or treatment of a disease associated with deregulated angiogenesis.

In particular, a therapeutically effective amount of each of the active ingredients of the COMBINATION OF THE INVENTION may be administered simultaneously or sequentially and in any order, and the components may be administered separately or as a fixed combination. For example, the delay of progression or treatment of diseases according to the invention may comprise (I) administration of the first active ingredient In free or pharmaceutically acceptable salt form and (ii) administration of the second active ingredient in free or pharmaceutically acceptable salt form, simultaneously or sequentially in any order, in jointly therapeutically effective amounts, preferably in synergistically effective amounts, e.g. in daily dosages corresponding to the amounts described herein. The individual active ingredients of the COMBINATION OF THE INVENTION can be administered separately at different times during the course of therapy or concurrently in divided or single combination forms. The instant invention is therefore to be understood as embracing all such regimes of simultaneous or alternating treatment and the term "administering" is to be Interpreted accordingly.

Unless stated otherwise, in the present disclosure organic radicals and compounds designated "lower" contain not more than 7, preferably not more than 4, carbon atoms.

Compounds which inhibit the VEGF receptor tyrosine kinase are those of formula I 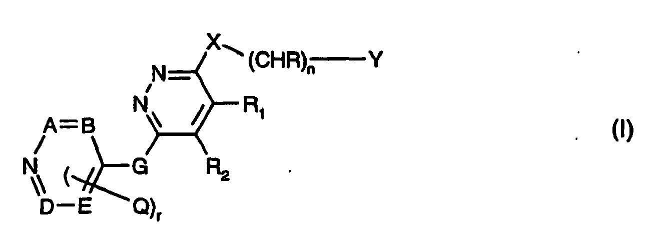 wherein
r is 0 to 2,
n is 0 to 2,
m is 0 to 4,
R₁ and R₂ (i) are lower alkyl or
   (ii) together form a bridge in subformula I* 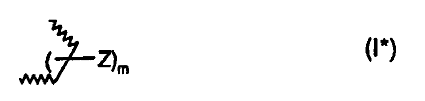 the binding being achieved via the two terminal carbon atoms, or
   (iii) together form a bridge in subformula I** 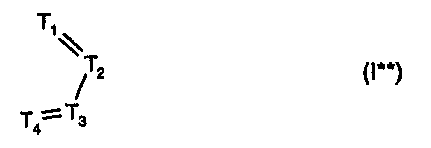
   wherein one or two of the ring members T₁, T₂, T₃ and T₄ are nitrogen, and the others are in each case CH, and the binding is achieved via T₁ and T₄;
A, B, D, and E are, independently of one another, N or CH, with the stipulation that not more than 2 of these radicals are N;
G is lower alkylene, lower alkylene substituted by acyloxy or hydroxy, -CH₂-O-, -CH₂-S-, -CH₂-NH-, oxa (-O-), thia (-S-), or imino (-NH-);
Q is lower alkyl;
R is H or lower alkyl;
X is imino, oxa, or thia;
Y is aryl, pyridyl, or unsubstituted or substituted cycloalkyl; and
Z is amino, mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl-lower alkylthio, alkylphenylthio, phenylsulfonyl, phenyl-lower alkylsulfinyl or alkylphenylsulfinyl, substituents Z being the same or different from one another if more than 1 radical Z is present;
and wherein the bonds characterized, if present, by a wavy line are either single or double bonds;
or an N-oxide of the defined compound, wherein 1 or more N atoms carry an oxygen atom; with the stipulation that, if Y is pyridyl or unsubstituted cycloalkyl, X is imino, and the remaining radicals are as defined, G is selected from the group comprising lower alkylene, -CH₂-O-, -CH₂-S-, oxa and thia;
and their pharmaceutically acceptable salts.

The radicals and symbols as used in the definition of a compound of formula I have the meanings as disclosed in WO 98/35958

The compound of formula I are preferably administered to the patient on a twice daily schedule.

The term "PTK787" as used herein means a VEGF receptor tyrosine inhibitor of formula I wherein r, n and m are each 0, R₁ and R₂ together form a bridge of subformula I*, A, B, D and E are each CH, G is methylene, X is imino, Y is 4-chlorophenyl, and the bonds characterized by a wavy line are double bonds.

The VEGF receptor tyrosine inhibitor of formula I of the present invention is PTK787. Most preferably, PTK787 is employed in the form of its succinate salt.

Compounds which inhibit the EGF receptor tyrosine kinase are 7H-pyrrolo[2,3-d]pyrimidine derivatives of formula IV 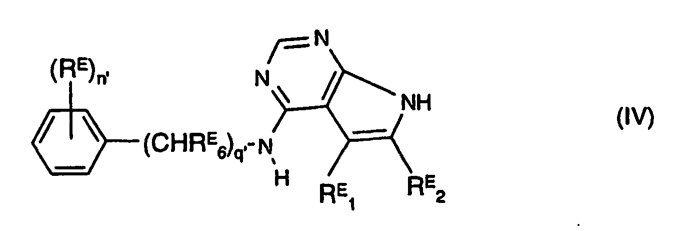 wherein
q' is 0 or 1,
n' is from 1 to 3 when q' is 0, or n' is from 0 to 3 when q' is 1,
R^{E} is halogen, lower alkyl, hydroxy, lower alkanoyloxy, lower alkoxy, carboxy, lower alkoxycarbonyl, carbamoyl, N-lower alkyl-carbamoyl, N,N-di-lower alkyl-carbamoyl, cyano, amino, lower alkanoylamino, lower alkylamino, N,N-di-lower alkylamino or trifluoromethyl, it being possible when several radicals R^{E} are present in the molecule for those radicals to be identical or different,
a) R^{E}₁, and R^{E}₂ are each independently of the other
α) phenyl substituted by carbamoyl-methoxy, carboxy-methoxy, benzyloxycarbonylmethoxy, lower alkoxycarbonyl-methoxy, phenyl, amino, lower alkanoylamino, lower alkylamino, N,N-di-lower alkylamino, hydroxy, lower alkanoyloxy, carboxy, lower alkoxycarbonyl, carbamoyl, N-lower alkyl-carbamoyl, N,N-di-lower alkyl-carbamoyl, cyano or by nitro;
β) hydrogen under the proviso that R^{E}₁ and R^{E}₂ cannot represent hydrogen at the same time;
γ) unsubstituted or halo- or lower alkyl-substituted pyridyl;
δ) N-benzyl-pyridinium-2-yl; naphthyl; cyano; carboxy; lower alkoxycarbonyl; carbamoyl; N-lower alkyl-carbamoyl; N,N-di-lower alkyl-carbamoyl; N-benzylcarbamoyl; formyl; lower alkanoyl; lower alkenyl; lower alkenyloxy; or
ε) lower alkyl substituted by
εα) halogen, amino, lower alkylamino, piperazino, di-lower alkylamino,
εβ) phonylamino that is unsubstituted or substituted in the phenyl moiety by halogen, lower alkyl, hydroxy, lower alkanoyloxy, lower alkoxy, carboxy, lower alkoxycarbonyl, carbamoyl, N-lower alkyl-carbamoyl, N,N-di-lower alkyl-carbamoyl, cyano, amino, lower alkanoylamino, lower alkylamino, N,N-di-lower alkylamino or by trifluoromethyl, εγ) hydroxy, lower alkoxy, cyano, carboxy, lower alkoxycarbonyl, carbamoyl, N-lower alkyl-carbamoyl, N,N-di-lower alkyl-carbamoyl, mercapto or
εδ) by a radical of the formula R^{E}₃-S(O)_{m'}- wherein R^{E}₃ is lower alkyl and m' is 0, 1 or 2, or
b) when q' is 0, one of the radicals R^{E}₁ and R^{E}₂ is unsubstituted lower alkyl or unsubstituted phenyl and the other of the radicals R^{E}₁ and R^{E}₂ has one of the meanings given above in paragraph a) with the exception of hydrogen, or
c) when q' is 1, R^{E}₁ and R^{E}₂ are each independently of the other unsubstituted phenyl or have one of the meanings given above in paragraph a), and
R^{E}₆ is hydrogen, lower alkyl, lower alkoxycarbonyl, carbamoyl, N-lower alkyl-carbamoyl or N,N-di-lower alkyl-carbamoyl,
and to the salts thereof.

The radicals and symbols as used in the definition of a compound of formula IV have the meanings as disclosed in WO 97/02266

The term "PKI166" as used herein means a EGF receptor tyrosine inhibitor of formula IV wherein q' is 1, n' is 0, R^{E}₁ is hydrogen, R^{E}₂ is phenyl substituted by 4-hydroxy, and R^{E}₆ is methyl.

One EGF receptor tyrosine inhibitor of formula IV of the present invention is PKI166.

PKI166 is preferably administered to the human subject less frequently than on a daily basis. In particular, the present invention relates to a treatment regimen whereby over at least a three week period, the EGF receptor tyrosine inhibitor PKI166 is administered on only about 40% to about 71% of the days. In such embodiment, specifically, the present invention relates to a method of treating a human subject with PKI166, which comprises administering such pyrimidine derivative to the human subject from three to five times in each seven day period for a period of three weeks or longer, more specifically, three or four times a week on alternate days for a period of three weeks or longer. In a specific embodiment, PKI166 is administered three times each week on alternate days, for example, on Monday, Wednesday and Friday of each week, for at least three weeks. Preferably, such dosage regimen is carried out through at least four or more weeks, for example 4, 5, 6, 7 or 8 weeks. Alternatively, PKI166 is administered daily for a period of one to three weeks, e.g. two weeks, followed by a period of one to three weeks, e.g. two weeks without administering the compound to the patient.

The other EGF receptor tyrosine inhibitor of formula IV of the present invention is a compound of formula IV, wherein q' is 1, n' is 0, R^{E}₁ is hydrogen, R^{E}₂ is phenyl substituted by CH₃-CH₂-CO-NH-, and R^{E}₆ is methyl.

In the first embodiment of the invention the first active ingredient is a compound which inhibits the VEGF receptor tyrosine kinase, viz. PTK787, and the second active ingredient is a compound which inhibits the EGF receptor tyrosine kinase, viz. PKI166.

In one preferred embodiment of the invention, the COMBINATION OF THE INVENTION is used for the treatment of cancer of the colon and generally the GI tract, glioma, renal cancer or cancer of the prostate, especially hormone refractory prostate cancer.

The COMBINATION OF THE INVENTION can further comprise additional active ingredients, e.g. an antineoplastic agent selected from the group consisting of aromatase Inhibitors, antiestrogens, topoisomerase I inhibitors, topoisomerase II inhibitors, microtubule active agents, alkylating agents, antineoplastic antimetabolites, platin compounds, gonadorelin agonists, anti-androgens and bisphosphonates.

The term "antineoplastic antimetabolites" includes, but is not limited to 5-fluorouracil, capecitabine, gemcitabine, methotrexate and edatrexate. Capecitabine can be administered, e.g., in the form as it is marketed, e.g. under the trademark XELODA^{™}. Gemcitabine can be administered, e.g., in the form as it is marketed, e.g. under the trademark GEMZAR^{™}.

The term "microtubule active agents" relates to microtubule stabilizing and microtubule destabilizing agents including, but not limited to the taxanes paclitaxel and docetaxel, the vinca alkaloids, e.g., vinblastine, especially vinblastine sulfate, vincristine especially vincristine sulfate, and vinorelbine, discodermolide and epothilones. Docetaxel can be administered, e.g., in the form as it is marketed, e.g. under the trademark TAXOTERE^{™}. Vinblastine sulfate can be administered, e.g., in the form as it is marketed, e.g. under the trademark VINBLASTIN R.P.^{™}. Vincristine sulfate can be administered, e.g., in the form as it is marketed, e.g. under the trademark FARMISTIN^{™}. Discodermolide can be obtained, e.g., as disclosed in US 5,010,099.

In one preferred embodiment of the invention a combination consisting of PTK787, PKI166 and XELODA^{™} is employed for the treatment of a solid tumor disease, especially glioma or colorectal cancer.

In another preferred embodiment of the invention a combination comprising PTK787, PKI166 and a taxane, e.g. paclitaxel or docetaxel, is employed for the treatment of a solid tumor disease, especially hormone resistant prostate cancer.

Moreover, the present invention relates to a treatment of a warm-blooded animal, in particular a human, having a proliferative disease comprising administering to the animal a COMBINATION OF THE INVENTION comprising a first active ingredient which is a vasculostatic compound and a second active ingredient which decrease the activity of the EGF, in a quantity which is jointly therapeutically effective against a disease associated with deregulated angiogenesis and in which the compounds can also be present in the form of their pharmaceutically acceptable salts.

In one preferred embodiment of such a treatment a warm-blooded animal said combination is administered to said mammal serially or simultaneously with radiation therapy.

Additionally, the present invention relates to the use of a a vasculostatic compound in combination with a compound which decreases the activity of the EGF. Furthermore, the present invention relates to the use of a a vasculostatic compound for the preparation of a medicament for the delay of progression or treatment of a disease associated with deregulated angiogenesis to be used in combination with a compound which decreases the activity of the EGF and to the use of a compound which decreases the activity of the EGF for the preparation of a medicament for the delay of progression or treatment of a disease associated with deregulated angiogenesis to be used in combination with a vasculostatic compound.

The effective dosage of each of the active ingredients employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This Involves a consideration of the distribution, equilibrium, and elimination of the active Ingredients.

If the the warm-blooded animal is a human, the dosage of a compound of formula I is preferably in the range of 150 to 4000, more preferably 200 to 2000, and most preferably 250 to 1000, mg/day, in the case of an adult patient.

In case of a compound of formula IV the dosage is preferably in the range of 50 to 700, more preferably 100 to 500, and most preferably 150 to 300, mg/day.

5-Fluorouracil may be administered to a human in a dosage range varying from 50 to 1000 mg/m²day, e.g. 500 mg/m²day.

Capecitabine may be administered to a human in a dosage range varying from 10 to 1000 mg/m²day.

Gemcitabine hydrochloride may be administered to a human in a dosage range varying from 1000 mg/week.

Methotrexate may be administered to a human in a dosage range varying from 5 to 500 mg/m²day.

Paclitaxel may be administered to a human in a dosage range varying from 50 to 300 mg/m²day.

Docetaxel may be administered to a human in a dosage range varying from 25 to 100 mg/m²day.

The following Examples illustrate the invention described above.

### Example 1: Treatment of mouse breast carcinoma induced by transfection of breast epithelial cells with neuT (transgenic organ tumor model)

The dual treatment with PTK787 in the form of its succinate salt (active ingredient 1, Example 62 of WO 98/35958) and an EGF receptor tyrosine inhibitor of formula IV wherein q' is 1, n' is 0, R^{E}₁ is hydrogen, R^{E}₂ is 3-acetylamino phenyl and R^{E}₆ is methyl (active Ingredient 2, Example 22e of WO 97/02266) is performed on a genetically engineered tumor model, which uses "transgenic" organs in normal mice. *Neu*T (the point mutated rat homolog of erbB-2) transfected HC11 epithelial mouse mammary epithelial cells are transplanted Into the gland-free mammary fat pad (cleared fat-pad) of the fourth mammary gland of female BALB/c mice according to an established method (DeOme, Faulkin, et al., Cancer Res. 19: 515-520, 1959). Within 4 to 6 weeks, the transplanted oncogene-transfected mammary epithelial cells develop breast tumors. Tumors are focal and heterogeneous in morphology, and oncogene and other molecular marker expression. Tumors grow rapidly and most of the animals develop breast tumors bilaterally after transplantation. The animals are allocated randomly to three different treatment groups. A first group is treated with 100 mg/kg of active ingredient 1 dosed once per day alone; a second group is treated with 100 mg/kg of active ingredient 2 dosed once per day alone; and a third group is treated with the combination of both active ingredients dosed with 100 mg/kg once per day. Treatment with active ingredient 1 alone results in 7% (1/14) regression, 21 % (2/14) tumors with stable disease and 78 % (11/14) tumors without response to the treatment with the VEGF receptor tyrosine inhibitor. The treatment with active ingredient 2 alone results in 46% (6/13) tumors with regression, 30 % (4/13) tumors show stable disease, whereas 23 % (3/13) tumors show no response to the EGF receptor tyrosine inhibitor. Dual treatment with both active ingredients results in 82 % (9/11) tumors with regression, 9 % (1/11) tumors with stable disease and 9 % (1/11) tumor with no response to the dual treatment.

### Example 2: DU145 prostate carcinoma human cell lines grown i.d. in nude mice

DU145 prostate carcinoma human cell lines are grown i.d. in nude mice. Tumor cell (10⁶) are injected intradermally (i.d.) on the left and right flank of nude mice. Treatment with compounds is started after 25-32 days when tumors reach a size of 80-100 mm². At this time animals are sorted into groups with equivalent mean and range of tumor sizes. Treatment is then randomized to the different groups. Tumor size is measured with calipers on a weekly basis.

Example 2.1 (comparative): After 4 weeks of treatment, 50 mg/kg po/day of PTK787 (active ingredient 1) reduces the tumor growth by 74%. 50 mg/kg po/day of the selective EGF receptor tyrosine kinase inhibitor as described in Example 1 of WO 96/33980 (active ingredient 2) reduces tumor growth by 91%. The maximum effects of either agent given alone in this model is stable disease. Simultaneous treatment with both active ingredients (50 mg/kg po/day of each active ingredient) results in tumor regression (25% reduction compared to intial tumor weight).

Example 2.2: After 2 weeks of treatment, 50 mg/kg po/day of PTK787 (active ingredient 1) reduces the tumor growth by 41%. 50 mg/kg po/day of the selective EGF receptor tyrosine kinase inhibitor PKI166 reduces tumor growth by 44%. Simultaneous treatment with both active ingredients (50 mg/kg po/day of each active Ingredient) reduces the tumor growth by 74%.

### Example 3: A431 human cervix carcinoma cell lines grown in nude mice

A431 human cervix carcinoma cell lines are injected subcutaneously on the back of athymic nude mice. Tumor growth is monitored daily by measuring perpendicular diameters. Treatment is started when the tumors reach a size of at least 0.175 cm³. At this time animals are sorted into groups with equivalent mean and range of tumor sizes. Treatment is then randomized to the different groups. Tumor size is measured with calipers on a weekly basis. The first group receives simultaneously 50 mg/kg po/day of PTK787 (active ingredient 1) and 50 mg/kg po/day of the selective EGF receptor tyrosine kinase inhibitor PKI166. The second group receives 50 mg/kg po/day of PTK787 (active ingredient 1) together with a daily, locoregional applied dose of 3 Gy on four consecutive days using an X-ray unit at 0.7 Gy/min about 30 minutes after the application of the compound PTK787. The third group receives simultaneously 50 mg/kg po/day of PTK787 (active ingredient 1) and 50 mg/kg po/day of the selective EGF receptor tyrosine kinase inhibitor PKI166 together with a daily, locoregional applied dose of 3 Gy on four consecutive days using an X-ray unit at 0.7 Gy/min about 30 minutes after the application of the compounds.

### Example 4: Clinical study design I

A human patient suffering from renal cell cancer is treated for a period of 16 weeks in 4 cycles consisting of administration of 600 mg of PKI166 daily for two weeks followed by 2 weeks without administering the drug. Additionally, PTK787 is administered twice daily, with a total daily dose of 300 mg. The tumor volume is measured by magnetic resonance imaging every 28 days.

### Example 5: Clinical study design II

A human patient suffering from renal cell cancer is treated for a period of 16 weeks in 4 cycles consisting of administration of 450 mg of PKI166 daily for two weeks followed by 2 weeks without administering the drug. Additionally, PTK787 is administered twice daily, with a total daily dose of 500 mg. The tumor volume is measured by magnetic resonance imaging every 28 days.

The Examples clearly demonstrate that the COMBINATION OF THE INVENTION exceed the anti-tumor effect of either active ingredient given as a single drug.

## Claims

1. A combination which comprises as a first active ingredient PTK787 and as a second active ingredient PKI166, a 7H-pyrrolo[2,3-d]pyrimidine derivative of formula IV 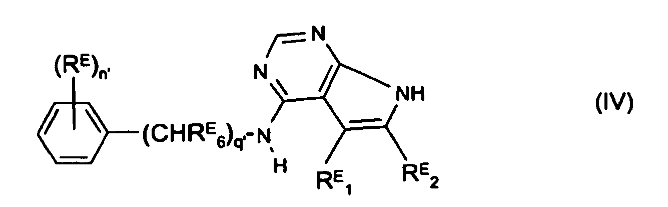 wherein
q' is 1, n' is 0, R^{E}₁ is hydrogen, R^{E}₂ is phenyl substituted by 4-hydroxy, and R^{E}₆ is methyl;
in which the active ingredients are present in each case in free form or in the form of a pharmaceutically acceptable salt and optionally at least one pharmaceutically acceptable carrier; for simultaneous, separate or sequential use.

2. A combination which comprises as a first active ingredient PTK787 and as a second active ingredient a 7H-pyrrolo[2,3-d]pyrimidine derivative of formula IV 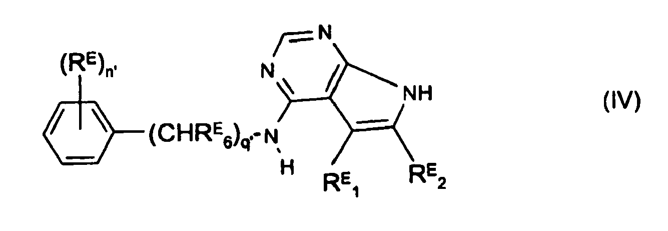 wherein
q' is 1, n' is 0, R^{E}₁ is hydrogen, R^{E}₂ is phenyl substituted by CH₃-CH₂-CO-NH-, and R^{E}₆ is methyl;
in which the active ingredients are present in each case in free form or in the form of a pharmaceutically acceptable salt and optionally at least one pharmaceutically acceptable carrier; for simultaneous, separate or sequential use.

3. Combination according to any one of claims 1 or 2 for simultaneous, separate or sequential use in the delay of progression or treatment of a disease associated with deregulated angiogenesis.

4. Combination according to claim 3 wherein the disease associated with deregulated angiogenesis is a proliferative disease.

5. Combination according to any one of claims 1 to 4 comprising further an antineoplastic agent selected from the group consisting of aromatase inhibitors, antiestrogens, topoisomerase I inhibitors, topoisomerase II inhibitors, microtubule active agents, alkylating agents, antineoplastic antimetabolites, platin compounds, gonadorelin agonists, anti-androgens and bisphosphonates.

6. Combination according to claim 5 consisting of PTK787, PKI166 and XELODA^{™}.

7. Combination according to claim 5 consisting of PTK787, PKI166 and a taxane.

8. A pharmaceutical composition comprising a quantity, which is jointly therapeutically effective against a disease associated with deregulated angiogenesis, of a pharmaceutical combination according to any one of claims 1 to 7 and at least one pharmaceutically acceptable carrier.

9. Use of a pharmaceutical combination according to any one of claims 1 to 7 for the preparation of a medicament for the delay of progression or treatment of a disease associated with deregulated angiogenesis.

## Patentansprüche

1. Kombination, die umfasst als ersten Wirkstoff PTK787 und als zweiten Wirkstoff PKI166, ein 7H-Pyrrolo[2,3-d]pyrimidinderivat der Formel IV 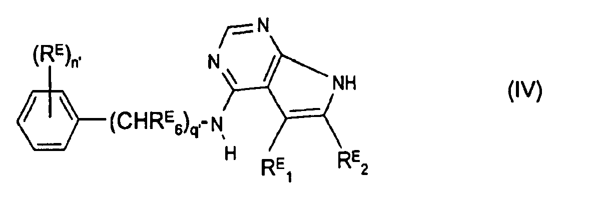 worin
q' für 1 steht, n' für 0 steht, R^{E}₁ für Wasserstoff steht, R^{E}₂ für Phenyl steht, das mit 4-Hydroxy substituiert ist und R^{E}₆ für Methyl steht,
worin die Wirkstoffe jeweils in freier Form oder in Form eines pharmazeutisch annehmbaren Salzes vorliegen und optional zumindest einen pharmazeutisch annehmbaren Träger zur simultanen, separaten oder sequenziellen Verwendung.

2. Kombination, die umfasst als ersten Wirkstoff PTK787 und als zweiten Wirkstoff ein 7H-Pyrrolo[2,3-d]-pyrimidinderivat der Formel IV 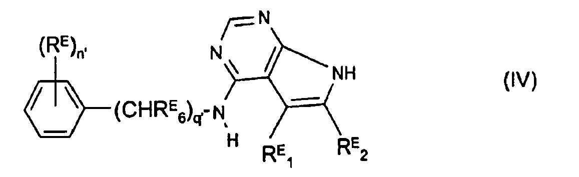 worin q' für 1 steht, n' für 0 steht, R^{E}₁ für Wasserstoff steht, R^{E}₂ für Phenyl steht, das durch CH₃-CH₂-CO-NH- substituiert ist und R^{E}₆ für Methyl steht,
worin die Wirkstoffe jeweils in freier Form oder in Form eines pharmazeutisch annehmbaren Salzes vorliegen und zumindest einen pharmazeutisch annehmbaren Träger zur simultanen, separaten oder sequenziellen Verwendung.

3. Kombination nach einem der Ansprüche 1 oder 2 zur simultanen, getrennten oder sequenziellen Verwendung bei der Verzögerung der Progression oder Behandlung einer Erkrankung, die mit einer deregulierten Angiogenese assoziiert ist.

4. Kombination nach Anspruch 3, worin die Erkrankung, die mit einer deregulierten Angiogenese assoziiert ist, eine proliferative Erkrankung ist.

5. Kombination nach einem der Ansprüche 1 bis 4, die ferner ein antineoplastisches Mittel umfasst, das aus der Gruppe ausgewählt ist, die besteht aus Aromataseinhibitoren, Antiöstrogenen, Topoisomerase I Inhibitoren, Topoisomerase II Inhibitoren, mikrotubulären Wirkstoffen, Alkylierungsmitteln, antineoplastischen Antimetaboliten, Platinverbindungen, Gonadorelinagonisten, Antiandrogenen und Bisphosphonaten.

6. Kombination nach Anspruch 5, die aus PTK787, PKI166 und XELODA® besteht.

7. Kombination nach Anspruch 5, die aus PTK787, PKI166 und einem Taxan besteht.

8. Pharmazeutische Zusammensetzung, die eine Menge einer pharmazeutischen Kombination nach einem der Ansprüche 1 bis 7 und zumindest einen pharmazeutisch annehmbaren Träger umfasst, die zusammen therapeutisch gegen eine Erkrankung wirksam ist, die mit deregulierter Angiogenese assoziiert ist.

9. Verwendung einer pharmazeutischen Kombination nach einem der Ansprüche 1 bis 7 zur Herstellung eines Arzneimittels zur Verzögerung der Progression oder Behandlung einer Erkrankung, die mit deregulierter Angiogenese assoziiert ist.

## Revendications

1. Combinaison comprenant, en tant que premier ingrédient actif, PTK787 et, en tant que second ingrédient actif, PKI166, un dérivé de 7H-pyrrolo[2,3-d]pyrimidine de formule IV 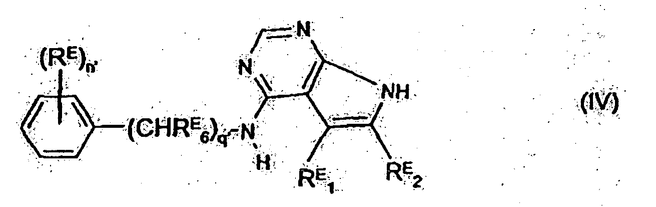 dans laquelle,
q' est 1, n' est 0, R^{E}₁ est hydrogène, R^{E}₂ est phényle substitué par 4-hydroxy, et R^{E}₆ est méthyle ;
**caractérisé en ce que** les ingrédients actifs sont présents, dans chacun des cas, sous forme libre ou sous forme d'un sel pharmaceutiquement acceptable et comprenant, éventuellement, au moins un véhicule pharmaceutiquement acceptable ; destinée à une utilisation simultanée, séparée ou séquencée.

2. Combinaison comprenant, en tant que premier ingrédient actif, PTK787 et, en tant que second ingrédient actif, un dérivé de 7H-pyrrolo[2,3-d] pyrimidine de formule IV 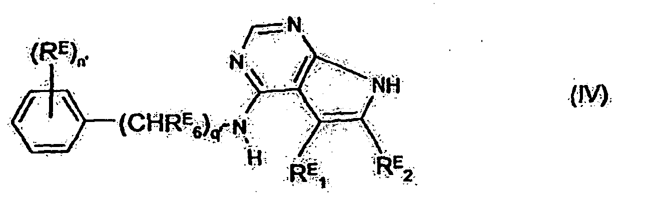 dans laquelle,
q' est 1, n' est 0, R^{E}₁ est hydrogène, R^{E}₂ est phényle substitué par CH₃-CH₂-CO-NH-, et R^{E}₆ est méthyle ;
**caractérisé en ce que** les ingrédients actifs sont présents, dans chacun des cas, sous forme libre ou sous forme d'un sel pharmaceutiquement acceptable et comprenant, éventuellement, au moins un véhicule pharmaceutiquement acceptable ; destinée à une utilisation simultanée, séparée ou séquencée.

3. Combinaison selon l'une quelconque des revendications 1 ou 2 destinée à une utilisation simultanée, séparée ou séquencée en vue de ralentir la progression ou de traiter une maladie associée à un dérèglement de l'angiogenèse.

4. Combinaison selon la revendication 3, **caractérisée en ce que** la maladie associée à un dérèglement de l'angiogenèse est une maladie proliférative.

5. Combinaison selon l'une quelconque des revendications 1 à 4 comprenant également un agent anti-néoplasique sélectionné à partir du groupe composé d'inhibiteurs de l'aromatase, d'anti-oestrogènes, d'inhibiteurs de la topoisomérase I, d'inhibiteurs de la topoisomérase II, d'agents activant la microtubule, d'agents alkylants, d'antimétabolites anti-néoplasiques, de composés platines, d'agonistes de la gonadoréline, d'anti-androgènes et de bisphosphonates.

6. Combinaison selon la revendication 5 comprenant du PTK787, du PKI166 et du XELODA^{™}.

7. Combinaison selon la revendication 5 comprenant du PTK787, du PKI166 et un taxane.

8. Composition pharmaceutique comprenant une quantité, qui est également thérapeutiquement efficace à l'encontre d'une maladie associée à l'angiogenèse, d'une combinaison pharmaceutique selon l'une quelconque des revendications 1 à 7 et au moins un véhicule pharmaceutiquement acceptable.

9. Utilisation d'une combinaison pharmaceutique selon l'une quelconque des revendications 1 à 7 dans la préparation d'un médicament destiné à ralentir la progression ou à traiter une maladie associée à un dérèglement de l'angiogenèse.
